# EUROPEAN PATENT APPLICATION

(11) **EP 2 335 768 A2**
(43) Date of publication of application: **22.06.2011**
(21) Application number: 09811175.0
(22) Date of filing: 12.06.2009
(51) Int. Cl.: A61M 25/10

(54) **INFLATABLE BALLOON CATHETER DEVICE AND METHOD FOR DYNAMIC REGULATION OF VENOUS RETURN**

(30) Priority: 02.09.2008 US 202617
(71) Applicant: Herrera Cedeño, José, Bogota N-A (CO)
(72) Inventor: Herrera Cedeño, José, Bogota N-A (CO)
(74) Representative: Martin Santos, Victoria Sofia
(86) International application number: PCT/IB2009/052506
(87) International publication number: WO 2010/026497

(57) **Abstract**

This invention relates to hydro-mechanical devices to regulate the venous return flow in the human circulatory system. Specifically, it refers to a combined catheter/cyclic occlusion inflatable balloon to regulate the venous return which can be adapted to its location space in the inferior vena cava, near the right entrance of atriun which comprises means to its retention inside such vessel as well as its ability to modify volume according to patient's needs.

## Description

### BACKGROUND OF THE INVENTION

It has been known for a long time that patients suffering congestive heart failure have an increase in the venous return to the heart (pre-load increase) and severe pulmonary congestion, so that it is determinant to diminish the venous return to obtain improvements both the pulmonary congestion symptoms and the congestive heart failure. A first attempt to alleviate this disease was using bleedings and tourniquets in the lower extremities in order to reduce such increased venous return (pre-load reduction) to improve such congestive symptomatology.

On later years and for reducing such pre-load the use of mercurial diuretics became common. More recently, it was proposed the use of strong loop diuretics such as Furosemide and Bumetanid which permit to obtain a quick reduction on the venous return. However these drugs have deleterious effects related to the patient renal function such as hydro-electrolytic disorders and renal damages.

As the problems with the increased pre-load continued, the use of nitrid and nitrates was considered due to these compounds produce a venous bed dilatation, causing a reduction on the venous return and symptom improvements for a short time. Nevertheless and due to tolerance troubles exhibited by this type of medicaments, their effects lose after a few months and besides it provides collateral effects as headaches in a great number of patients which makes difficult to use these medicines during extended periods of time.

As a result of the introduction of the receptor blockers of the aldosterone (spironolactone) it was possible to diminish the hydrosaline retention as well as the venous return.

Afterwards: A new pharmacological alternative was introduced, consisting of the drugs that block the formation of angiotensin, producing a decrease of the venous return, improving the quality of life of patients with heart failure.

Later the use of beta blockers of the last generation carvedilol type was recommended, this produced significant benefits in patients with heart failure being the treatment of choice for a high number of patients with said illness.

V1-V2 receptor antagonists of the neuro-hormonal arginine - vasopressin axis (type Tovaltan, Conivaptan) were used to produce a reduction of the corporal water and a decrease on the venous return as well as the typical symptoms of pulmonary congestion for a short period.

The stem cells usage for the treatment of the congestive heart failure was considered at the yearly meeting of the American Heart Association in 2007 and a conclusion from this meeting was that such usage provides light improvements on the ejection fraction of the left ventricle and better results are obtained only when preload is diminished through diuretic administration. It is evident that a good pre-load handling is very important to improve the left ventricle performance and relieve pulmonary congestion in patients with congestive heart failure.

Lastly and in extreme cases when all known therapeutic measures fail, mechanical methods are tried to reduce the venous return by removing corporal water such as dialysis and ultra-filtration. Such methods improve the symptoms which are common on the congestive heart failure but during short periods after that the body returns to the prior state the increased of venous return and the pulmonary congestion increased.

### OBJECTS OF THE INVENTION

An object of the invention is to solve in an efficient and effective way the above explained troubles through a mechanical regulation of the venous return on patients suffering congestive heart failure.

Another object of the invention is to provide, for such regulation, a catheter carrying an inflatable balloon which can be adjusted on its volume and externally filled to carry out such adjust by way of a physiological solution.

An additional object of the invention is to provide, once placed and fixed said catheter/balloon through sub-clavia and right atriun at the proximal portion of the inferior vena cava and up to a near site from the inferior vena cava outlet, an instantaneous dynamic cyclic occlusion occurs, in order to obtain a regulation on the flow running from said inferior vena cava toward the right atriun.

Another object of the invention is to provide a balloon able to be fixed or anchored at the selected place into the vena cava by way of a retractile screw.

### BRIEF DESCRIPTION OF THE DRAWINGS

Fig. 1 is a general and schematic view of the balloon according to the invention which comprises amplified details from the fixing element and said inflatable balloon, wherein a) is a longitudinal section view; b) is a transversal section view; c) is a sagittal section view and d) is an amplified section of the b) view.
Fig. 2 is a view showing the balloon placed in the vena cava when is in the expiration phase.
Fig. 3 is a similar view of Fig 4 with said vena cava when is in the inspiration phase.
Fig. 4 is a similar view of Fig 2 only for indicating the return of the vein to the expiration phase.

It is a purpose of the present invention, as stated before, to provide a method and a device to regulation, mechanically, the increased venous return (or pre-load) on patients suffering congestive heart failure and such method is based on the application of a catheter having an inflatable adjustable balloon, which can be filled with a physiological solution from outside of the body. Such catheter and the balloon are introduced via sub-clavia vein, passing on right atriun up to the proximal portion of the inferior vena cava where is fixed on the rear wall thereof. Thus, the balloon stays anchored very near from the inferior vena cava outlet where is greater the inspiratory collapse. Successively it is inflated up to a point, previously determined, which corresponds to the vein occlusion requirements. The inflated balloon produces a cyclic and instantaneous occlusion according to a continued succession of inspiratory collapses (inspiratory collapse time: one second) and expirations. Thus, a regulated flow from the inferior vena cava toward the right atriun is obtained.

Therefore, the right atriun and right ventricle load conditions are reduced. This cycle is repeated on every inspiration preventing to increase the flow volume produced on each inspiration, therefore, a favorable instantaneous volume decreased is obtained and consequently, a filling pressure reduction in right and left ventricles. As a result, an advantageous effect over the pulmonary congestive symptoms is obtained as well as over both ventricles diastolic diameters and an improvement on the ventricular ejection.

This model of instantaneous and dynamic flow regulation is further advantageous in the sense that the balloon can be easily implanted by one skilled in the art, it is possible to make adjustments *in situ* without inconveniences and does not require external energy to operate since the flow regulation is supplied by the own patient body when he/she breaths. Moreover, it can be withdrawn without troubles if required by emptying its content and from outside.

The proposed operating way by the present inventor to adjust the balloon and to obtain an adequate regulation effect on each case or patient situation can be explained, in general terms through the following examples and the corresponding illustrative figures attached to this description.

### Example 1

After localizing the patient inferior vena cava by way of bidimensional echocardiography, in sub-costal position, and measured the vena cava diameter during expiration (*v*.g., 2 cm), it is measured the collapse thereof which commonly is 50%, that is, a 1 cm diameter reduction.

Under these conditions, the balloon has to be inflated until 1 cm to complete the remaining space obtaining now a 100% occlusion during one second (inspiration).

### Example 2

In a case where the inferior vena cava sizes 2.0 cm and the inspiratory collapse is 40% a diameter contraction in such blood vessel should be near about 1.26 cm. Then, such balloon should have to be inflated in the same measure to complete the missing diameter thus obtaining a 100% occlusion as desired during inspiration. It achieves a dynamic flow regulation. In the subsequent expiration step the vena cava returns to its normal diameter, allowing the flow.

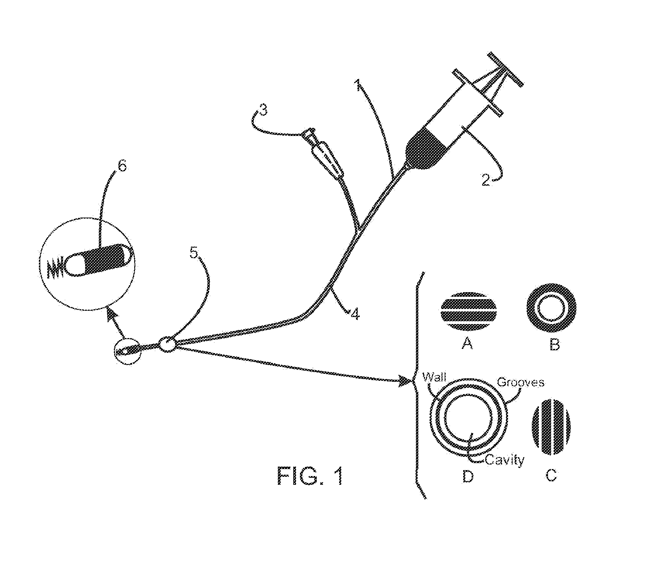

Referring now to figures accompanying this description, it can be seen that the catheter/balloon assembly 1 comprises a two-ways deviation 4 toward its proximal end, where one branch leads to a cylindrical body 2 having a piston, to inject a liquid (a saline or glucose solution) toward the inner space of the balloon 5 and the other branch is a sheath 3 to lead a wire connected to a distal screw 6 in order to fix the catheter on inferior vena cava wall. The injection body 2 comprises an inner check-valve (not shown) to prevent the injected liquid into balloon 5 returns. As can be seen from a detail A in Figure 1 such balloon 5 is configured as an ovoid to soften the blood current flow. Additionally, it comprises surface slits guided in parallel to the longitudinal axis.

The balloon 5 is able to be inflated from 1 cm to 2 cm on its larger diameter when filled with the above mentioned saline or glucose solution. It comprises a wall 1 mm thick where the external slits (0,5 mm deep and 1 mm wide) are foreseen to facilitate the balloon expansion when injected the saline or glucose solution through the catheter.

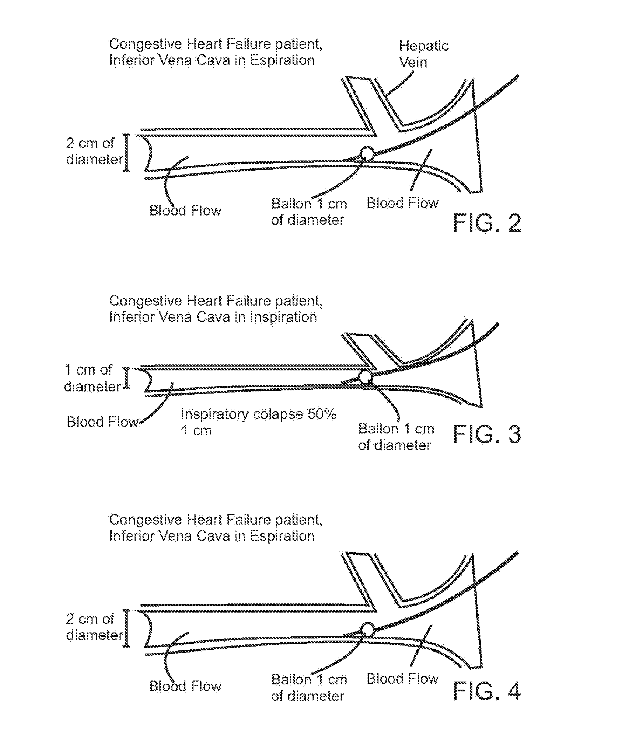

The schematic Figures 2 and 3 show how operates the method proposed herein, in the assumption that is applied to a patient suffering congestive heart failure. Fig. 2 shows an inferior vena cava portion which corresponds to the confluence zone where join said vena cava and hepatic vein. The vena cava is in the expiration step and at this stage is expanded 2 cm in diameter. The balloon 5 (diameter = 1 cm) previously inserted into vena cava, is located near the hepatic vein outlet, due to is the main inspiratory collapse space. It can be seen that at this expiration condition the blood flow way is essentially free and without the inserted balloon may affect said passage in any manner.

Figure 3 represents the vena cava at the inspiration phase having 1 cm in diameter, which now is the same diameter than the balloon and producing the vessel self-occlusion (which is the pursued purpose) during about one second (inspiratory collapse time). When this time is over, the vein returns to the expiration phase allowing again the flow of blood, as illustrated in Figure 2. The venous return or pre-load diminishes due to the instantaneous flow reduction as well as the decreasing on the filling pressure over right and left ventricles as previously explained.

## Claims

1. A method and a catheter device comprising an inflatable balloon for a dynamic regulation of the venous return or pre-load, wherein said method comprises introducing via sub-clavia vein, right auricle and inferior vena cava proximal portion and near the hepatic vein outlet, a two-way catheter carrying an inflatable balloon which can be filled and inflated later with a saline or glucose solution forming an occlusion into said inferior vena cava during the inspiration phase.

2. A method, according to claim 1, wherein de two-way catheter comprises two branches wherein the first one allows to fill such balloon and the second one permits to reach and manage, through a wire, a catheter fixing screw on the vena cava wall.

3. A method, according to claim 1, wherein said balloon once placed at the vena cava, near the hepatic vein outlet or maximum collapse site, is inflated through the filling branch up to reaching the same diameter corresponding to the vena cava diameter when is in inspiration or collapse phase, forming an occlusion against the blood flow which goes toward right auricle.

4. A method, according to claims 1 and 3, wherein once overcame the inspiration phase and reached the expiration phase, the blood flow remains free to continue due to the normal vena cava expansion.

5. The device, according to claim 1, wherein the balloon is made using an appropriate flexible material, shaped as an ovoid, having longitudinally aligned slits in order to facilitate the balloon wall expansion as well as the ability to increase its spherical volume between 1 and 2 cm³.
